(19) Europäisches Patentamt
European Patent Office
Office européen des brevets

(11) Publication number: 0 175 558 A1

(12) # EUROPEAN PATENT APPLICATION

(21) Application number: 85306533.2

(22) Date of filing: 13.09.85

(51) Int. Cl.[4]: C 07 C 29/136, C 07 C 31/08, C 07 C 67/00, C 07 C 69/14, C 07 C 31/10

(30) Priority: 17.09.84 US 650947

(43) Date of publication of application: 26.03.86 Bulletin 86/13

(84) Designated Contracting States: BE DE FR GB IT NL

(71) Applicant: THE HALCON SD GROUP, INC., 2 Park Avenue, New York, N.Y. 10016 (US)

(72) Inventor: Moy, David, 460 Van Emburgh Avenue, Ridgewood New Jersey 07450 (US)

(74) Representative: Cropp, John Anthony David et al, MATHYS & SQUIRE 10 Fleet Street, London, EC4Y 1AY (GB)

(54) **Process for the vapor phase hydrogenation of carboxylic acids to esters and alcohols.**

(57) The vapor phase formation of carboxylic acid alcohols and/or esters such as ethanol and ethyl acetate from the corresponding mono and di-functional carboxylic acid, such as acetic acid, in the presence of a copper oxide-metal oxide supported catalyst, such as $CuO/ZnAl_2O_4$.

EP 0 175 558 A1

## BACKGROUND OF THE INVENTION

### Field of the Invention

This invention relates to the hydrogenation of carboxylic acids to the corresponding alcohols and esters, and more particularly, to a low temperature, low pressure, vapor phase hydrogenation of mono and di-functional carboxylic acids to the corresponding alcohol and/or ester, in the presence of a copper oxide supported catalyst.

### DESCRIPTION OF THE PRIOR ART

Carboxylic acids are a well known class of organic compounds possessing a wide variety of both bulk industrial and diverse specialty usages. The hydrogenation of carboxylic acids and their derivatives to alcohols and/or esters has been attempted in the art, usually in liquid phase reactions. U.S. patent 2,093,159 discloses the catalytic hydrogenation of esters of carboxylic acids over catalytic substances such as copper, nickel, silver, zinc, cadmium, lead or cobalt and activating substances including certain transition metals, alkali metals, alkaline earth metals and rare earth metals.

U.S. patent 4,398,039 discloses a process for the vapor phase hydrogenation of carboxylic acids to the corresponding alcohols, in the presence of steam and a catalyst comprising the mixed oxides of ruthenium, at least one of cobalt or nickel, and, optionally, one of cadmium, zinc, copper, iron and several other metals. This process, however, produces high conversions only at higher temperatures and pressures than are considered desirable.

U.K. patent application 2,116,552 discloses a process for the hydrogenolysis of a carboxylic acid ester, comprising contacting the ester and hydrogen with a catalyst formed from a reduced mixture of copper oxide and zinc oxide, at a temperature from about 75-300°C and at pressures ranging from about 0.1-100 $Kg/cm^2$ absolute.

It is an object of this invention to provide a process for the preparation of selected alcohols and/or esters

in high yields and high conversions from the corresponding carboxylic acid through the catalytic hydrogenation reaction of the selected acid.

It is another object of this invention to provide a process in which selected carboxylic acids are hydrogenated to the corresponding alcohols and/or esters in a vapor phase reaction occurring under moderate temperatures and super-atmospheric pressures in which the hydrogenation catalyst activity and resulting product yield are substantial.

It is still another object of the invention to provide a catalyst which is easily replaceable, economically feasible and as active as those of the prior art.

It is yet another object of the invention to provide a process for the preparation of ethanol and/or ethyl acetate from acetic acid in the vapor phase under moderate conditions of temperature and pressure in a catalytic reaction featuring high reactant conversions and product yields.

SUMMARY OF THE INVENTION

Accordingly, these and other objects have been achieved by the discovery of a novel process and accompanying copper oxide supported catalyst for the vapor phase formation of mono and di-functional carboxylic acid alcohols and/or esters containing about 1 to 20 carbon atoms from the corresponding mono and di-functional carboxylic acids, i.e., mono and di-functional aryl, aryl-aliphatic and aliphatic carboxylic acids of 1 to 10 carbons, comprising contacting the carboxylic acid with hydrogen gas, preferably in amounts ranging from about 500:1 to 3:1 moles $H_2$ per mole of carboxylic acid, most preferably about 20:1 to 75:1, under reaction temperatures of about 175-350°C and pressures of about 50-5000 psig, in the presence of a copper oxide-metal oxide supported, e.g., $ZnAl_2O_4$, ZnO, or $SiO_2$, catalyst, with the preferred embodiment represented by the following stoichiometric formula:

$$Cu_aZn_bAl_cO_d$$

wherein $a = 0.1 - 0.9$

$b = 0.1 - 1.0$

$c = 2b$

$d$ = the number of oxygens determined by the valence requirements of the other elements.

DETAILED DESCRIPTION OF THE INVENTION

Carboxylic acids which are suitable feedstocks for the process of the invention can comprise both mono and di-functional acids, preferably aryl, mixed aryl-aliphatic and aliphatic carboxylic acids containing from one to about ten carbon atoms. The process is particularly suitable for the hydrogenation of saturated carboxylic acids of the formula

$$R-\underset{\underset{O}{\|}}{C}-OH$$

wherein R represents a $C_1$ to $C_{10}$ branched or straight chain alkyl aryl, or mixed alkyl-aryl moiety. Preferred carboxylic acids which can be converted to their corresponding alcohol and/or ester include acetic, n-butyric, isobutyric, propionic, lactic, the valeric acids and the like. In the broadest embodiment of the invention, substantially any volatile organic acid or acid mixture that has a boiling point below about 250°C may be suitable for use.

In the broadest embodiment, the class of suitable catalysts can encompass a wide range of copper oxides in combination with other oxides, which can be chosen from a variety of metal oxide, e.g., non-acidic supports and the like. Preferably, suitable compositions involve CuO with metal oxide support, i.e., $ZnAl_2O_4$, ZnO, $SiO_2$, and the like, with $ZnAl_2O_4$ the most preferred. The preferred catalyst support can operate over a wide variety of proportions, as is seen in the following empirical formula:

$Cu_aZn_bAl_cO_d$

wherein a = 0.1 - 0.9
b = 0.1 - 1.0
c = 2b
d = the number of oxygens determined by the valence requirements of the other elements. In $CuO/ZnAl_2O_4$ supported catalysts, the catalyst can comprise from about 1 to 80 wt. % copper oxide, most preferably, about 5% to 45 wt. % copper oxide.

The chemical reaction occurring during the process is that of a carboxylic acid reacting with hydrogen under appropriate conditions to form an alcohol and/or ester product. This vapor phase hydrogenation may be carried out in any suitable catalytic reactor, such as an autoclave, a fluidized bed or, more preferably, a fixed bed tube reactor wherein the acid is first heated to the vaporous or gaseous state before being contacted with a suitable volume of hydrogen at effective pressures and temperatures in the presence of the selected hydrogenation catalyst. Since hydrogenation reactions are generally exothermic, a suitable cooling means can be employed to control and maintain the temperature within the desired range. The vapor phase reaction products produced by the reactor are recovered and suitably treated in conventional manner, such as by condensation and subsequent fractional distillation, so as to effect the separation of the products from the reactants.

The copper oxide supported catalysts can be prepared by conventional methods known in the art; most preferably, the preferred catalysts are produced by forming a $ZnAl_2O_4$ spinel which is extruded and then depositing CuO by impregnation or by dry powder coating. The catalysts used in the process are preferably at least partially reduced before being utilized in the hydrogenation reaction. This prereduction may be carried out by contacting the catalyst with the reductant, preferably hydrogen gas, at a temperature ranging from about 50°C to 300°C, preferably about 200°C to 300°C.

The hydrogenation reaction is conducted in the vapor phase at temperatures ranging from about 175°C to about 300°C, preferably about 250°C to 275°C. The reaction pressure can generally range between about 50 to about 5000 psig, preferably about 100 to 1000 psig. Hydrogen gas is introduced into the reaction zone in excess with respect to the carboxylic acid in a molar ratio of at least 3:1, preferably about 75:1 to 20:1, and up to ratios of about 500:1. The carboxylic acid can be introduced if desired into the reaction zone in the presence of a suitable carrier, or solvent.

Although it is preferred to proceed without employing a solvent, operable solvents for the hydrogenation process of the instant invention are the hydrocarbons and ethers, with selected alcohols also suitable in some instances.

As mentioned above, it is preferred to employ the catalyst in the presence of a metal oxide support. Preferred supports beside the most preferred $ZnAl_2O_4$ are $SiO_2$, ZnO, Silica-alumina, alumina, oxides of groups IIA, IIIA, VIA metals, as well as various mixtures thereof.

By following the process of this invention, a clean, simple, one step, low temperature and low pressure reaction can be obtained whereby the carboxylic acid is converted to the desired alcohol and/or ester, depending upon the precise reaction conditions shown. Conditions favoring production of the alcohol are the higher pressure and temperature environments, while the conditions favoring production of the ester are the corresponding lower temperature and pressure ranges for suitable operation.

The vapor phase hydrogenation catalysts employed in the process of this invention may be subject to a rapid loss of catalytic activity or degradation, due to several factors and conditions which must be carefully controlled, reduced or eliminated in order to make the resulting process economical. For example, catalyst poisoning and the resulting loss of catalytic activity therefrom, which is a

substantial problem for copper oxide based catalysts, appears to be due to the uncontrolled or improper dispersion of the exothermic heat of reaction, which can cause hydrogenolysis of the resulting alcohol products, together with assisting in the formation of salts and polythermic esters which, when deposited on the catalyst, substantially decrease the hydrogenation activity thereof. Additional catalytic strength has been found to be extremely important and it is highly desirable that the finished catalyst retain its strength over a long period of exposure to reaction conditions.

EXAMPLES

Example 1. Preparation of $CuO/ZnAl_2O_4$

Equimolar amounts of ZnO and $Al_2O_3$ (formed by neutralization of the nitrates with $Na_2CO_3$) were intimately mixed, pasted and extruded (1/4" x 1/16"). The extrudates were calcined at 950-1000°C for 6 hours to give a BET surface area of 42 $m^2$/gm and a porosity of about 0.6 cc/gm.

Four hundred grams of extrudate were then soaked in a solution of 116 gms $Cu(NO_3)_2 \cdot 3H_2O$ in 240 cc water for 1 hour. The excess liquid was removed by filtration and the particles were dried overnight at 105°C. The impregnated extrudates were then soaked with an 0.3 M $Na_2CO_3$ solution for 10 minutes to convert the copper compounds to the basic copper carbonate. The particles were then thoroughly washed with excess water (to pH of about 7), dried overnight at 105°C, and calcined in air at 350°C for 4 hours. The loading after calcination was determined to be 5.9% (as CuO).

Example 2. Preparation of CuO/ZnO on Silica Gel

One hundred grams of silica gel (Davison, Grade 58, porosity 1.1 cc/gm), 3-12 mesh was soaked in a solution of 0.8 M $Cu(NO_3)_2$ and 0.25 M in $Zn(NO_3)_2$. The particles were filtered and dried at 105°C. They were then soaked in a 1.2 M $Na_2CO_3$ solution for 0.25 hours to convert the nitrates to the mixed hydroxides/carbonates. The resulting catalyst was washed thoroughly with water (to pH of about 7). The particles were dried and calcined at 350°C

for 4 hours. The loading of CuO was determined to be 6.9%; the ZnO loading was 2.0%.

Example 3

Copper nitrate and zinc nitrate were impregnated on gamma-alumina as in Example 2. The nitrates were converted to the oxides directly by calcination at 450°C in air for six hours. The resultant CuO and ZnO loadings were 9.4 and 4.7 wt. %, respectively, and the surface area of the catalyst was 230 $m^2$/gm.

Examples 4-10

The catalysts in Examples 1 and 2 were used to hydrogenolyze acetic acid in tubular stainless steel reactor (75 or 150 cc catalyst bed). The catalysts were activated at 300°C and 750 psig with an 0.5-5% mixture of $H_2$ in $N_2$. After activation the gas stream was switched to pure $H_2$.

The hydrogenolysis of acetic acid was carried out at the conditions indicated in Table 1. Pure acetic acid was pumped into the reactor and vaporized in a pre-heater zone. Product effluent analyses and off-gas analyses were carried out by gas chromatography.

Following the procedure set forth in Examples 4-10, the catalyst of Example 3 was activated and used for the hydrogenolysis of acetic, propionic and lactic acids to their corresponding alcohols/esters. The results are set forth in Examples 11-15 in Table I.

TABLE I

| Ex. | Catalyst | | Temp. (°C) | Press. (psig) | Feed (Acid) | Feed Rate (cc/hr) | $H_2$/Acid Mole Ratio | Conversion Acid | Selectivity | |
|---|---|---|---|---|---|---|---|---|---|---|
| | | | | | | | | | Ester | Alcohol |
| 4 | Ex.1, | 150 cc | 275 | 500 | Acetic | 12 | 72 | 100 | 0.6[A] | 99[B] |
| 5 | " | " | " | 750 | " | " | " | 100 | 0.4 | 99 |
| 6 | " | " | " | " | " | 10 | 60 | 100 | 1.0 | 99 |
| 7 | Ex.2, | 75 cc | 300 | 100 | | 10 | 25 | 13 | 41 | 36 |
| 8 | " | " | " | 300 | " | " | " | 23 | 60 | 42 |
| 9 | " | " | " | 500 | " | " | " | 38 | 59 | 39 |
| 10 | " | " | " | 750 | " | " | " | 50 | 60 | 38 |
| 11 | Ex.3 | 150 cc | 250 | 100 | | 15 | 77 | 95 | 5 | 87 |
| 12 | " | " | 280 | 100 | " | " | " | 100 | 1 | 88 |
| 13 | " | " | " | 300 | Propionic | " | 45 | 99 | 1[C] | 94[D] |
| 14 | " | " | " | 100 | " | " | 45 | 98 | 6 | 82 |
| 15 | " | " | 240 | 300 | Lactic | 10 | 199 | 99 | NA | 60[E] |

A = Ethyl Acetate
B = Ethanol
C = n-Propyl Propionate
D = 1-Propanol
E = Propylene Glycol

CLAIMS

1. A process for the vapor phase formation of carboxylic acid alcohols and/or esters containing 1 to 20 carbon atoms from the corresponding mono and di-functional carboxylic acids comprising:-

contacting the carboxylic acid with hydrogen gas under reaction temperatures between about 175°C to 350°C and pressures of about 50 to 5000 psig in the presence of a copper oxide-metal oxide supported catalyst.

2. A process as claimed in claim 1 wherein the corresponding mono and di-functional carboxylic acids are mono and di-functional aryl, aryl-aliphatic and aliphatic carboxylic acids of 1 to 10 carbons.

3. A process as claimed in claim 1 wherein the carboxylic acid is acetic acid.

4. A process as claimed in claim 1 wherein ethanol or ethyl acetate is produced from acetic acid.

5. A process as claimed in any one of claims 1 to 4 wherein the $H_2$/carboxylic acid molar ratio ranges from about 3:1 to 500:1 and preferably from about 20:1 to 75:1.

6. A process as claimed in any one of claims 1 to 5 wherein the reaction is carried out in the presence of a heterogeneous catalyst.

7. A process as claimed in any one of claims 1 to 6 wherein the catalyst comprises oxides of copper and zinc on a support.

8. A process as claimed in any one of claims 1 to 6 wherein the catalyst is a $CuO/ZnAl_2O_4$ supported composition.

9. A process as claimed in claim 8 wherein the $CuO/ZnAl_2O_4$ catalyst composition contains about 1-80 wt. % CuO.

10. A process as claimed in any one of claims 1 to 7 wherein the copper oxide catalyst can be represented by the formula:

$$Cu_aZn_bAl_cO_d$$

wherein a = 0.1 - 0.9

b = 0.1 - 1.0

c = 2b

d = the number of oxygens determined by the valence requirements of the other elements.

11. A process as claimed in any one of claims 1 to 9 wherein the pressure ranges from about 100 to 1000 psig.

12. A process as claimed in any one of claims 1 to 10 wherein the temperature ranges from about 250 to 275°C.

0175558

European Patent Office

# EUROPEAN SEARCH REPORT

Application number

EP 85 30 6533

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-1 154 221 (VEREINIGTE GLANZSTOFF-FABRIKEN AG)<br>* Page 1, left-hand column, paragraph 2 - page 1, right-hand column *<br>--- | 1 | C 07 C 29/136<br>C 07 C 31/08<br>C 07 C 67/00<br>C 07 C 69/14<br>C 07 C 31/10 |
| A | BE-A- 892 958 (DAVY McKEE)<br>* Page 6, line 7 - page 8, line 15; page 12, lines 4-25 *<br>----- | 1 | |
| | | | **TECHNICAL FIELDS SEARCHED (Int. Cl.4)** |
| | | | C 07 C 29/00<br>C 07 C 31/00<br>C 07 C 67/00<br>C 07 C 69/00 |

The present search report has been drawn up for all claims

| Place of search | Date of completion of the search | Examiner |
|---|---|---|
| THE HAGUE | 11-11-1985 | KINZINGER J.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82